# EUROPEAN PATENT APPLICATION

(11) **EP 4 779 649 A1**
(43) Date of publication of application: **22.07.2026**
(21) Application number: 24882611.7
(22) Date of filing: 14.08.2024
(51) Int. Cl.: G16H 50/20, G16H 10/60, G16H 70/00, G16H 50/30, G16H 20/60, G16H 20/30, A61B 5/00

(54) **ELECTRONIC DEVICE FOR PROVIDING MESSAGE ABOUT HEALTH CARE AND OPERATION METHOD THEREOF**

(30) Priority: 27.10.2023 KR 20230146095; 28.11.2023 KR 20230168229
(71) Applicant: Samsung Electronics Co., Ltd., Suwon-si, Gyeonggi-do 16677 (KR)
(72) Inventor: PARK, Byeongju, Suwon-si Gyeonggi-do 16677 (KR); YEO, Jaeyung, Suwon-si Gyeonggi-do 16677 (KR); KIM, Jaepil, Suwon-si Gyeonggi-do 16677 (KR); PARK, Jeongmin, Suwon-si Gyeonggi-do 16677 (KR); LEE, Myeongjin, Suwon-si Gyeonggi-do 16677 (KR); CHO, Sunghwan, Suwon-si Gyeonggi-do 16677 (KR)
(74) Representative: Gulde & Partner
(86) International application number: PCT/KR2024/012120
(87) International publication number: WO 2025/089582

(57) **Abstract**

According to an embodiment of the present invention, an electronic device may comprise: at least one processor; and a memory storing instructions. The instructions may be individually or collectively executed by the at least one processor to instruct the electronic device to generate a message for the health care of a user on the basis of state information about the user and first information. The instructions may be individually or collectively executed by the at least one processor to instruct the electronic device to determine whether the message is suitable for the health state of the user on the basis of second information and profile information about the user.

## Description

### TECHNICAL FIELD

Embodiments of the present disclosure relate to an electronic device for providing a message about health care and an operation method thereof.

### DESCRIPTION OF RELATED ART

Electronic devices including a function for providing health information have been widely distributed. An electronic device may provide health information to a user through a generative language model. The generative language model is an artificial intelligence model trained based on large-scale text data. The generative language model may be used for text generation and question answering. For example, the generative language model may include a large language model (LLM).

An electronic device may generate a response (e.g., a health care message) to a user's question. When a health care message is generated from large-scale text data (e.g., data related to general health information), the quality of the health care message may be determined depending on the quality of the large-scale text data. When the large-scale text data includes errors, the health care message may also include errors.

In addition, a general health care message may not consider an individual user's health condition and thus may include information that is inappropriate for the user.

The above information may be presented as the related art to help with the understanding of the disclosure. No arguments or decisions are raised to whether any of the above description is applicable as the prior art related to the present disclosure.

The technical goals to be achieved are not limited to those described above, and other technical goals not mentioned above are clearly understood by one of ordinary skill in the art from the following description.

### DISCLOSURE OF THE INVENTION

### TECHNICAL SOLUTIONS

According to an embodiment, an electronic device includes at least one processor and memory storing instructions. The instructions, when executed individually or collectively by the at least one processor, may cause the electronic device to generate, based on state information of a user and first information, a message about health care for the user. The instructions, when executed individually or collectively by the at least one processor, may cause the electronic device to determine, based on second information and profile information of the user, whether the message is appropriate for the health condition of the user. The first information may include general health-related information. The second information may include professional health-related information.

According to an embodiment, a method of operating an electronic device includes generating, based on state information of a user and first information, a message about health care for the user. The method may include determining, based on second information and profile information of the user, whether the message is appropriate for the health condition of the user. The first information may include general health-related information. The second information may include professional health-related information.

According to an embodiment, an electronic device includes at least one processor and memory storing instructions. The instructions, when executed individually or collectively by the at least one processor, may cause the electronic device to generate, based on state information of a user and health-related information, a message about health care for the user. The instructions, when executed individually or collectively by the at least one processor, may cause the electronic device to determine, based on profile information of the user, whether the message is appropriate for the health condition of the user.

According to an embodiment, a non-transitory computer-readable storage medium may store instructions that, when executed by a processor, cause the processor to perform the method.

### BRIEF DESCRIPTION OF THE DRAWINGS

FIG. 1 is a block diagram illustrating an electronic device in a network environment according to an embodiment.
FIG. 2 is a diagram illustrating an operation of an electronic device according to an embodiment.
FIG. 3 is a diagram illustrating an operation of a message generator according to an embodiment.
FIGS. 4A and 4B are diagrams illustrating an operation of a message filter according to an embodiment.
FIG. 5 illustrates an example of a flowchart of a method of providing a message about health care according to an embodiment.
FIG. 6 is a flowchart illustrating a method of generating a message about health care for a user according to an embodiment.
FIG. 7 is a diagram illustrating an operation of determining whether a message is appropriate for a health condition of a user, based on a reliability score, according to an embodiment.
FIG. 8 is a diagram illustrating an operation of determining whether a message is appropriate for a health condition of a user, based on an appropriateness score, according to an embodiment.
FIG. 9 is a diagram illustrating a message verified based on second information according to an embodiment.
FIG. 10 is a diagram illustrating a message verified based on profile information of a user according to an embodiment.
FIGS. 11A to 11C are diagrams illustrating a comparison between a verified message and an unverified message based on various profile information of a user according to an embodiment.
FIGS. 12A and 12B are diagrams illustrating a message through which profile information is provided to different users according to an embodiment.

### DETAILED DESCRIPTION

Hereinafter, the examples will be described in detail with reference to the accompanying drawings. When describing the embodiments with reference to the accompanying drawings, like reference numerals refer to like elements and a repeated description related thereto will be omitted.

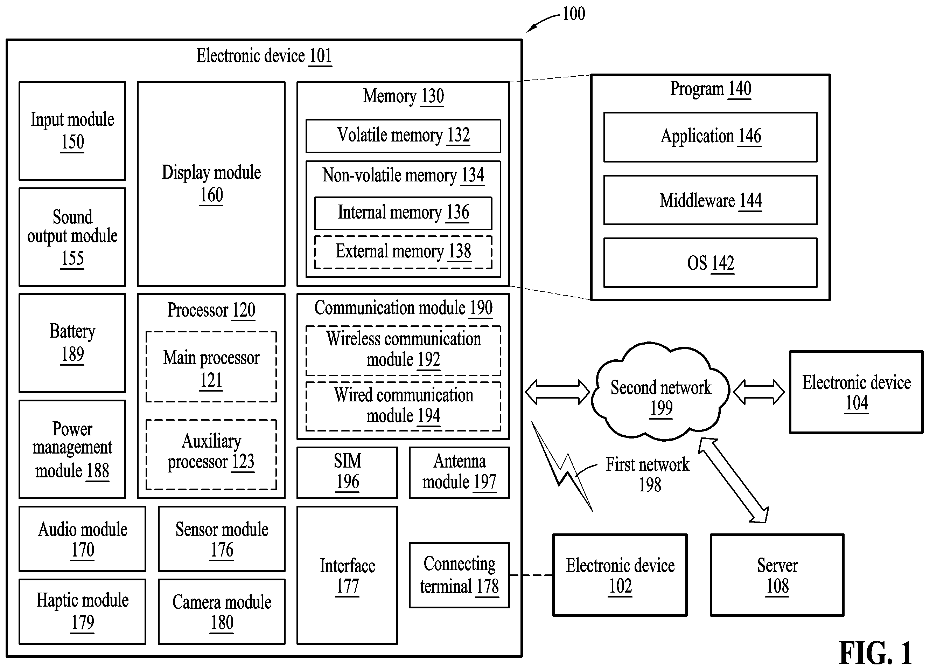

FIG. 1 is a block diagram illustrating an electronic device 101 in a network environment 100 according to an embodiment. Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, and a sensor module 176, an interface 177, a connecting terminal 178, a haptic module 179, a camera module 180, a power management module 188, a battery 189, a communication module 190, a subscriber identification module (SIM) 196, or an antenna module 197. In some examples, at least one (e.g., the connecting terminal 178) of the above components may be omitted from the electronic device 101, or one or more other components may be added to the electronic device 101. In some examples, some (e.g., the sensor module 176, the camera module 180, or the antenna module 197) of the components may be integrated as a single component (e.g., the display module 160).

The processor 120 may execute, for example, software (e.g., a program 140) to control at least one other component (e.g., a hardware or software component) of the electronic device 101 connected to the processor 120 and may perform various data processing or computation. According to an embodiment, as at least a portion of data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may be implemented as circuitry (e.g., processing circuitry), such as a system-on-chip (SoC) or an integrated circuit (IC). The processor 120 may include one or more processors. For example, the processor 120 may include a combination of one or more processors, such as a CPU, a GPU, a microprocessor unit (MPU), an AP, and a CP. According to an embodiment, the processor 120 may include a main processor 121 (e.g., a central processing unit (CPU) or an application processor (AP)) or an auxiliary processor 123 (e.g., a graphics processing unit (GPU), a neural processing unit (NPU), an image signal processor (ISP), a sensor hub processor, or a communication processor (CP)) that is operable independently of, or in conjunction with the main processor 121. For example, when the electronic device 101 includes the main processor 121 and the auxiliary processor 123, the auxiliary processor 123 may be adapted to consume less power than the main processor 121 or to be dedicated for a designated function. The auxiliary processor 123 may be implemented separately from the main processor 121 or as a part of the main processor 121.

The auxiliary processor 123 may control at least some of functions or states related to at least one (e.g., the display module 160, the sensor module 176, or the communication module 190) of the components of the electronic device 101, instead of the main processor 121 while the main processor 121 is in an inactive (e.g., sleep) state, or together with the main processor 121 while the main processor 121 is an active state (e.g., executing an application). According to one embodiment, the auxiliary processor 123 (e.g., an ISP or a CP) may be implemented as a portion of another component (e.g., the camera module 180 or the communication module 190) that is functionally related to the auxiliary processor 123. According to one embodiment, the auxiliary processor 123 (e.g., an NPU) may include a hardware structure specified for artificial intelligence (AI) model processing. The AI model may be generated by machine learning. Such learning may be performed by, for example, the electronic device 101 in which an AI model is executed, or performed via a separate server (e.g., the server 108). Learning algorithms may include, but are not limited to, for example, supervised learning, unsupervised learning, semi-supervised learning, or reinforcement learning. The AI model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may additionally or alternatively include a software structure other than the hardware structure.

The memory 130 may store various pieces of data used by at least one component (e.g., the processor 120 or the sensor module 176) of the electronic device 101. The various pieces of data may include, for example, software (e.g., the program 140) and input data or output data for a command related thereto. The memory 130 may include the volatile memory 132 or the non-volatile memory 134. According to an embodiment, the memory 130 may include one or more memories. The instructions stored in the memory 130 may be stored in one memory. The instructions stored in the memory 130 may be distributed and stored in a plurality of memories. According to an embodiment, the instructions stored in the memory 130, when executed individually or collectively by at least one processor (e.g., the main processor 121 and/or the auxiliary processor 123), may cause the electronic device 101 to perform one or more operations. For example, the instructions stored in the memory 130 may be executed by a single processor (e.g., the main processor 121 or an auxiliary processor 123, such as a CP), or by a plurality of processors (e.g., the main processor 121 and the auxiliary processor 123) operating cooperatively.

The program 140 may be stored as software in the memory 130, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for general purposes, such as playing multimedia or playing a recording. The receiver may be used to receive an incoming call. According to an embodiment, the receiver may be implemented separately from the speaker or as a part of the speaker.

The display module 160 may visually provide information to the outside (e.g., a user) of the electronic device 101. The display module 160 may include, for example, a display, a hologram device, or a projector and control circuitry to control a corresponding one of the display, the hologram device, and the projector. According to an embodiment, the display module 160 may include a touch sensor adapted to sense a touch, or a pressure sensor adapted to measure an intensity of a force incurred by the touch. The display module 160 may be implemented with, for example, a foldable structure and/or a rollable structure. For example, a size of a display screen of the display module 160 may be reduced when folded and expanded when unfolded.

The audio module 170 may convert a sound into an electrical signal and vice versa. According to an embodiment, the audio module 170 may obtain the sound via the input module 150 or output the sound via the sound output module 155 or an external electronic device (e.g., an electronic device 102 such as a speaker or a headphone) directly or wirelessly connected to the electronic device 101.

The sensor module 176 may detect an operational state (e.g., power or temperature) of the electronic device 101 or an environmental state (e.g., a state of a user) external to the electronic device 101, and generate an electric signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used by the electronic device 101 to couple with the external electronic device (e.g., the electronic device 102) directly (e.g., by wire) or wirelessly. According to an example embodiment, the interface 177 may include, for example, a high-definition multimedia interface (HDMI), a universal serial bus (USB) interface, a secure digital (SD) card interface, or an audio interface.

The connecting terminal 178 may include a connector via which the electronic device 101 may physically connect to an external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or an electrical stimulus which may be recognized by a user via his or her tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, ISPs, or flashes.

The power management module 188 may manage power supplied to the electronic device 101. According to an embodiment, the power management module 188 may be implemented as, for example, at least a part of a power management integrated circuit (PMIC).

The battery 189 may supply power to at least one component of the electronic device 101. According to an embodiment, the battery 189 may include, for example, a primary cell that is not rechargeable, a secondary cell that is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more CPs that are operable independently of the processor 120 (e.g., an AP) and that support direct (e.g., wired) communication or wireless communication. According to one embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module, or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multiple components (e.g., multiple chips) separate from each other. The wireless communication module 192 may identify and authenticate the electronic device 101 in a communication network, such as the first network 198 or the second network 199, using subscriber information (e.g., international mobile subscriber identity (IMSI)) stored in the SIM 196.

The wireless communication module 192 may support a 5G network after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., a mmWave band) to achieve, e.g., a high data transmission rate. The wireless communication module 192 may support various technologies for securing performance on a high-frequency band, such as, e.g., beamforming, massive multiple-input and multiple-output (MIMO), full dimensional MIMO (FD-MIMO), an array antenna, analog beamforming, or a large-scale antenna. The wireless communication module 192 may support various requirements specified in the electronic device 101, an external electronic device (e.g., the electronic device 104), or a network system (e.g., the second network 199). According to one embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., an external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in a communication network, such as the first network 198 or the second network 199, may be selected by, for example, the communication module 190 from the plurality of antennas. The signal or the power may be transmitted or received between the communication module 190 and the external electronic device via the at least one selected antenna. According to an embodiment, another component (e.g., a radio frequency integrated circuit (RFIC)) other than the radiating element may be additionally formed as a portion of the antenna module 197.

According to an embodiment, the antenna module 197 may form a mmWave antenna module. According to an embodiment, the mmWave antenna module may include a PCB, an RFIC disposed on a first surface (e.g., the bottom surface) of the PCB or adjacent to the first surface and capable of supporting a designated high-frequency band (e.g., the mmWave band), and a plurality of antennas (e.g., array antennas) disposed on a second surface (e.g., the top or a side surface) of the PCB, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 and 104 may be a device of a same type as, or a different type from, the electronic device 101. According to one embodiment, all or some of operations to be executed by the electronic device 101 may be executed at one or more external electronic devices (e.g., the external devices 102 and 104, and the server 108). For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request and may transfer an outcome of the performing to the electronic device 101. The electronic device 101 may provide the outcome, with or without further processing of the outcome, as at least part of a reply to the request. To that end, cloud computing, distributed computing, mobile edge computing (MEC), or client-server computing technology may be used, for example. The electronic device 101 may provide ultra-low-latency services using, e.g., distributed computing or MEC. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

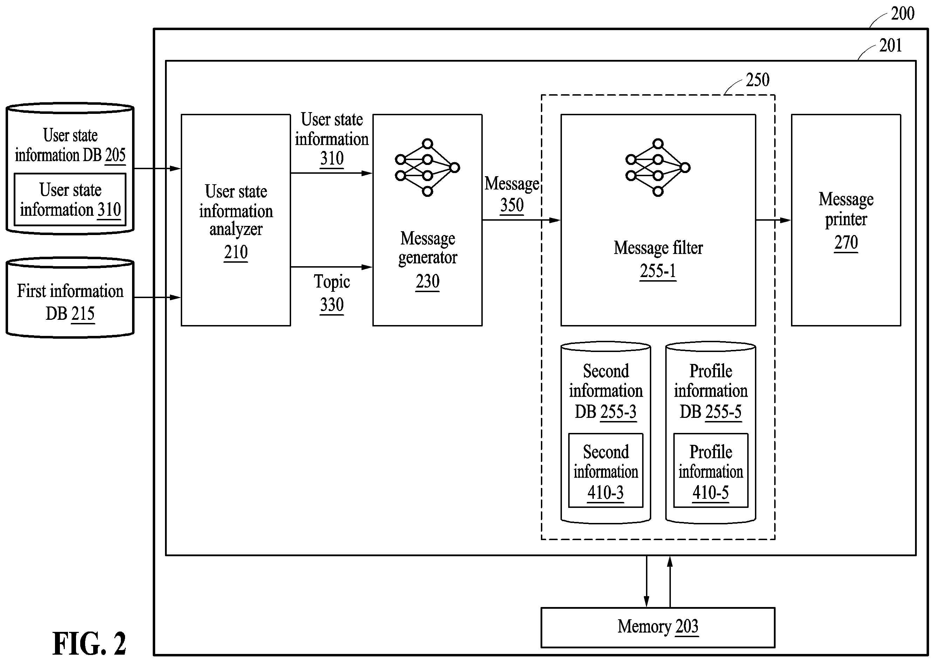

FIG. 2 is an example of a block diagram of an electronic device according to an embodiment.

Referring to FIG. 2, according to an embodiment, an electronic device 200 (e.g., the electronic device 101 of FIG. 1) may include a processor 201 (e.g., the processor 120 of FIG. 1) and memory 203 (e.g., the memory 130 of FIG. 1). A user state information analyzer 210, a message generator 230, a message verifier 250, and a message printer 270 may be executed by the processor 201 and may be implemented as one or more of program code including instructions that may be stored in the memory 203, an application, an algorithm, a routine, a set of instructions, or an AI training model. One or more of the user state information analyzer 210, the message generator 230, the message verifier 250, and the message printer 270 may be implemented in hardware and/or in a combination of hardware and software. The memory 203 may store one or more of a second information database (DB) 255-3 and a profile information DB 255-5, and in some embodiments, memory (not shown) separate from the memory 203 included in the electronic device 200 may store one or more of the second information DB 255-3 and the profile information DB 255-5.

According to an embodiment, the processor 201 may be implemented as circuitry (e.g., processing circuitry), such as a system-on-chip (SoC) or an integrated circuit (IC). The processor 201 may include one or more processors. For example, the processor 201 may include a combination of one or more processors, such as a CPU, a GPU, an MPU, an AP, and a CP. The instructions stored in the memory 203 may be executed by a single processor to cause the electronic device 200 to perform and/or control the operations of the electronic device 200 as described with reference to FIGS. 2 to 12B. The instructions stored in the memory 203 may be executed by a plurality of processors to cause the electronic device 200 to perform and/or control the operations of the electronic device 200 as described with reference to FIGS. 2 to 12B.

According to an embodiment, the memory 203 may include one or more memories. The instructions stored in the memory 203 may be stored in a single memory. The instructions stored in the memory 203 may be distributed and stored in a plurality of memories. The instructions stored in the memory 203 may be executed by the processor 201 to cause the electronic device 200 to perform and/or control the operations of the electronic device 200 as described with reference to FIGS. 2 to 12B.

According to an embodiment, the electronic device 200 may be implemented as at least one of smartphones, tablet personal computers (PCs), mobile phones, speakers (e.g., AI speakers), video phones, e-book readers, desktop PCs, laptop PCs, netbook computers, workstations, servers, personal digital assistants (PDAs), portable multimedia players (PMPs), MP3 players, mobile medical devices, cameras, or wearable devices.

According to an embodiment, a user state information DB 205 may be a DB that stores user state information 310. The user state information 310 may include information related to biometrics and/or lifestyle of a user. For example, the user state information 310 may include health information (e.g., a current health condition, symptoms, and discomfort of a user), lifestyle-related information (e.g., eating habits, mealtimes, sleep patterns, and daily activities), exercise records (e.g., an amount of daily activity, types of exercise, exercise durations, and an amount of calories burned during exercise), medical records (e.g., an electronic medical record (EMR) and an electronic health record (EHR)), and biometric information (e.g., information obtained from a sensor (e.g., a biometric sensor)). The biometric information may include heart rate, blood pressure, and step count.

According to an embodiment, a first information DB 215 may be a DB that stores first information. The first information may include general health-related information. For example, the first information may include information related to general health knowledge in daily life (e.g., nutrition, exercise, stress management, sleep, and hygiene). The first information DB 215 may be implemented external to the electronic device 200.

According to an embodiment, the user state information analyzer 210 may obtain (e.g., receive) the user state information 310 from the user state information DB 205. The user state information analyzer 210 may obtain (e.g., receive) first information from the first information DB 215. The user state information analyzer 210 may analyze the current state of the user based on the user state information 310 and/or the first information. The user state information analyzer 210 may determine a topic 330 of a message (e.g., a health care message) based on a change in the analyzed current state of the user. The topic 330 may correspond to the user state information 310. For example, the topic 330 may include, but is not limited to, a health information-related topic, a biometric information-related topic, a lifestyle-related topic, an exercise-related topic, and a medical record-related topic.

According to an embodiment, the user state information analyzer 210 may preprocess (and/or normalize) the user state information 310 obtained from the user state information DB 205 for analyzing the current state of the user.

According to an embodiment, the user state information analyzer 210 may determine, based on the preprocessed user state information, whether user state information (e.g., user state information before being preprocessed by the user state information analyzer 210) obtained from the user state information DB 205 has changed. The user state information analyzer 210 may select a priority based on a change in the user state information 310. When a plurality of user state information 310 has changed, the priority may be selected to generate a message corresponding to one of the changed user state information. For example, the priority may be selected in the order of biometric information, exercise records, lifestyle patterns, and medical records. However, the foregoing examples of priority are provided merely for purposes of describing the present disclosure, and the scope of the present disclosure is not to be construed as being limited thereto. The priority may be selected based on user input (e.g., user feedback regarding the priority).

According to an embodiment, the user state information analyzer 210 may determine the topic 330 necessary for providing health care to the user based on the selected priority when the user state information 310 has changed. When the plurality of user state information 310 has changed, the user state information analyzer 210 may determine the topic 330 corresponding to a change in user state information with a high priority. For example, when a change in biometric information (e.g., an increase in heart rate) and a change in a lifestyle pattern (e.g., a change in a sleep pattern) occur, the user state information analyzer 210 may determine the topic 330 corresponding to a change in biometric information with a high priority.

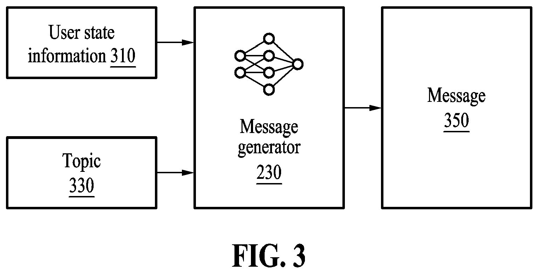

According to an embodiment, the message generator 230 may generate a message 350 (e.g., a message about health care for the user) based on the user state information 310 and the topic 330. The method of generating a message about health care for the user by the message generator 230 will be described in detail with reference to FIG. 3.

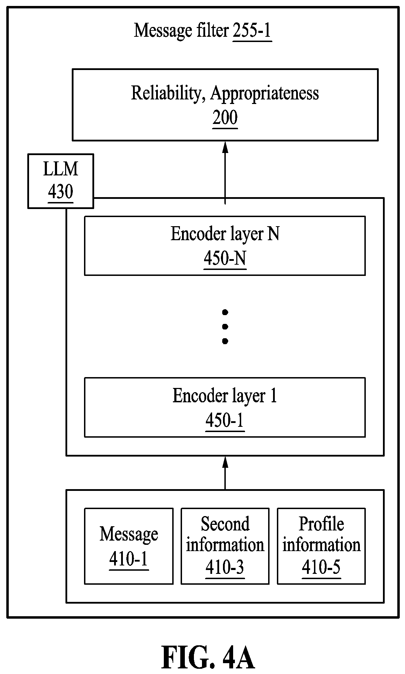

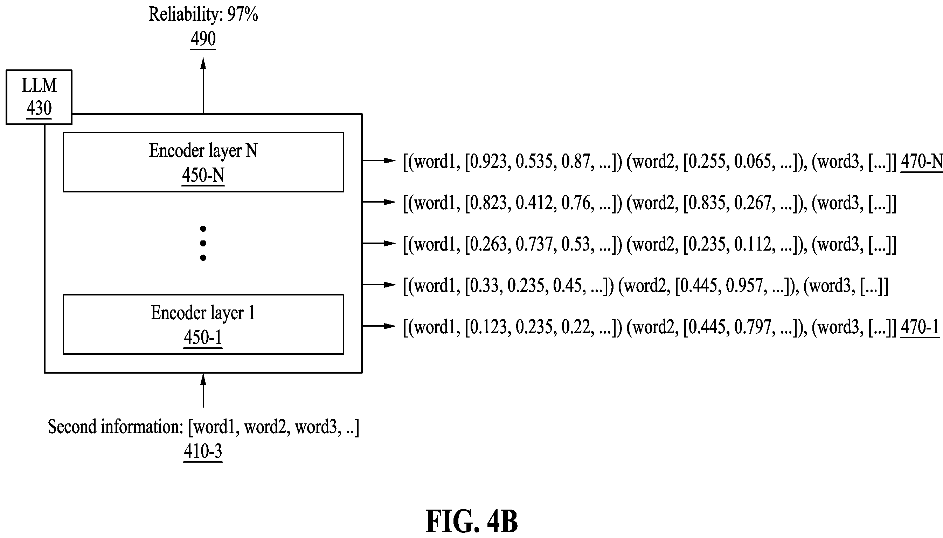

According to an embodiment, the message verifier 250 may verify the message 350 generated from the message generator 230. A message filter 255-1 may be included. The message filter 255-1 may verify the message 350 based on second information 410-3 stored in the second information DB 255-3 and/or profile information 410-5 of the user stored in the profile information DB 255-5. The method of verifying the message 350 by the message filter 255-1 will be described in detail with reference to FIGS. 4A and 4B.

According to an embodiment, the second information 410-3 may include professional health-related information. For example, the second information 410-3 may include medical literature and medical information.

According to an embodiment, the profile information 410-5 of the user may include information related to a specific health condition of the user. The profile information 410-5 of the user may include more specific information regarding the health condition of the user than the user state information 310. For example, the profile information 410-5 of the user may include basic user information (e.g., gender, age, and occupation), body information (e.g., height, weight, and body mass index (BMI)), and health condition information (e.g., presence or absence of underlying diseases, medication information, and health examination results).

According to an embodiment, the message printer 270 may include the display module 160 of FIG. 1. The message printer 270 may output a verified message. For example, the message printer 270 may display (or provide) the verified message to the user.

FIG. 3 is a diagram illustrating an operation of a message generator according to an embodiment.

Referring to FIG. 3, according to an embodiment, the message generator 230 may generate the message 350 about health care for a user based on the user state information 310 and the topic 330. The message generator 230 may include a generative language model (not shown). The generative language model (not shown) may include an AI model trained based on first information.

According to an embodiment, the message generator 230 may generate the message 350 based on a change in the user state information 310 corresponding to the topic 330.

According to an embodiment, the topic 330 may be a biometric information-related topic in response to an increase in the user's heart rate. The message generator 230 may generate the message 350 (e.g., a message such as "Your heart rate has been higher than usual. You may need some rest. Please relax your body and take slow, deep breaths.") based on biometric information (e.g., information indicating that the user's heart rate is increasing) and the topic 330.

According to an embodiment, the topic 330 may be an exercise record-related topic in response to a decrease in exercise duration. The message generator 230 may generate the message 350 (e.g., a message such as "Your recent exercise duration has been shorter than usual. Please increase your exercise time to help maintain your health.") based on an exercise record (e.g., information indicating that the user's exercise duration is decreasing) and the topic 330.

According to an embodiment, the topic 330 may be a lifestyle pattern-related topic in response to a change in the user's lifestyle pattern (e.g., when the user's mealtime is later than usual). The message generator 230 may generate the message 350 (e.g., a message such as "Your recent mealtime has been later than usual. Please adjust your mealtime to an earlier hour.") based on lifestyle pattern information (e.g., information indicating that the user's mealtime has become later) and the topic 330.

According to an embodiment, the topic 330 may be a medical record-related topic in response to a change in a medical record (or clinical record) (e.g., when the user's blood glucose level increases). The message generator 230 may generate the message 350 (e.g., a message such as "Your recent medical record indicates that your blood glucose level has been higher than usual. Please maintain regular meals and exercise to help manage your blood glucose level.") based on a medical record (e.g., a medical record indicating an increase in the user's blood glucose level) and the topic 330.

FIG. 4A is a diagram illustrating an operation of a message filter according to an embodiment.

Referring to FIG. 4A, according to an embodiment, the message filter 255-1 may verify a message 410-1 based on the second information 410-3 and the profile information 410-5 of a user. The message 410-1 may be obtained from the message generator 230 of FIG. 2. For example, the message filter 255-1 may determine whether the message 410-1 is appropriate for the user's health condition based on the second information 410-3 and the profile information 410-5 of the user.

According to an embodiment, the message filter 255-1 may include a large language model (LLM) 430. The LLM 430 may include a plurality of encoder layers (Encoder layer 1 450-1 to Encoder layer N 450-N). The LLM 430 may be used to verify the message 410-1.

According to an embodiment, the message filter 255-1 may calculate a reliability score of the message 410-1 based on the second information 410-3. The reliability score is a numerical score indicating the degree to which the message 410-1 is reliable with respect to medical facts.

According to an embodiment, the message filter 255-1 may calculate an appropriateness score of the message 410-1 based on the profile information 410-5 of the user. The appropriateness score is a numerical score indicating the degree to which the message 410-1 is appropriate for the health condition of an individual user.

According to an embodiment, the message filter 255-1 may verify the message 410-1 based on the reliability score and/or the appropriateness score. For example, the message filter 255-1 may determine whether the message 410-1 is appropriate for the user's health condition based on the reliability score and/or the appropriateness score.

Hereinafter, the method of calculating a reliability score will be described in detail with reference to FIG. 4B.

Referring to FIG. 4B, according to an embodiment, the LLM 430 may calculate a reliability 490 (e.g., a reliability score) through a plurality of encoder layers (e.g., Encoder layer 1 450-1 to Encoder layer N 450-N) based on the second information 410-3. The second information 410-3 may include a plurality of words (e.g., word 1, word 2, and/or word 3). The second information 410-3 may be independently assigned to each of the encoder layers (e.g., Encoder layer 1 450-1 to Encoder layer N 450-N). Each encoder layer may obtain context information (e.g., the relationship, semantics, and/or structure between the second information 410-3 and the message 410-1) by comparing the second information 410-3 with the message 410-1. Each encoder layer may vectorize the assigned second information 410-3 and compare it with the message 410-1. According to an embodiment, Encoder layer 1 450-1 may obtain a word vector 470-1 representing the second information 410-3. Encoder layer 1 450-1 may set (or assign) a weight to each component (e.g., corresponding to a word included in the second information 410-3) of the word vector 470-1. Encoder layer 1 450-1 may obtain the relationship between the second information 410-3 and the message 410-1 by comparing (or analyzing) the weighted word vector 470-1 with the message 410-1. The relationship between the second information 410-3 and the message 410-1 may include the result of comparing the message 410-1 with sentences (e.g., a set of one or more words) included in the second information 410-3.

According to an embodiment, each encoder layer may calculate the reliability 490 of the message 410-1 based on the context information obtained between the second information 410-3 and the message 410-1. According to an embodiment, Encoder layer 1 450-1 may determine which words in the second information 410-3 are similar to the message 410-1, based on context information (e.g., the result of comparing the message 410-1 with sentences included in the second information 410-3). The LLM 430 may obtain the reliability 490 of the message 410-1 by summing similarities (e.g., the degree of similarity between words in the second information 410-3 and the message 410-1) generated (or calculated) by each encoder layer. Similarity may be calculated using Euclidean distance, cosine similarity, and/or Jaccard similarity. However, the method of calculating similarity is not limited to the foregoing examples.

According to an embodiment, each encoder layer may determine which component (e.g., a component corresponding to one or more of a plurality of words included in the second information 410-3) of a word vector (e.g., a word vector corresponding to each encoder layer among word vectors 470-1 to 470-N) contributes most to calculation of the reliability of the message 410-1. Each encoder layer may estimate from which sentence in the second information 410-3 the reliability of the message 410-1 is calculated by setting (or resetting) a weight to a component that contributes most to calculation of the reliability.

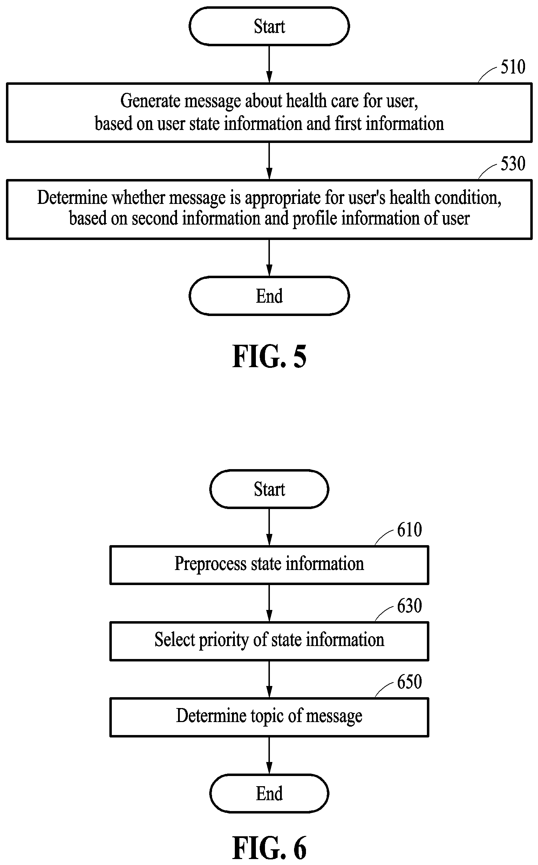

FIG. 5 is a flowchart illustrating a method of providing a message about health care according to an embodiment.

Referring to FIG. 5, according to an embodiment, operations 510 to 530 may be performed sequentially, but not necessarily. For example, the order of operations 510 to 530 may be changed, and at least two operations may be performed in parallel.

In operation 510, the electronic device 200 may generate a message (e.g., the message 350 of FIG. 3) about health care for a user based on user state information (e.g., the user state information 310 of FIG. 3) and first information.

In operation 530, the electronic device 200 may determine whether the message (e.g., the message 410-1 of FIG. 4A) is appropriate for the user's health condition based on second information (e.g., the second information 410-3 of FIG. 4A) and profile information of the user (e.g., the profile information 410-5 of FIG. 4A). The electronic device 200 may determine whether the message 410-1 is appropriate for the user's health condition by comparing a reliability score and/or an appropriateness score with a threshold.

According to an embodiment, when the message 410-1 is appropriate for the user's health condition, the electronic device 200 may provide the message 410-1 to the user. For example, the electronic device 200 may provide the message 410-1 to the user in response to determining that the message 410-1 is appropriate for the user's health condition.

According to an embodiment, when the message 410-1 is inappropriate for the user's health condition, the electronic device 200 may regenerate the message 410-1. For example, the electronic device 200 may output feedback in response to determining that the message 410-1 is inappropriate for the user's health condition. The feedback may include a request to regenerate a message. The electronic device 200 may regenerate a message based on feedback. The electronic device 200 may determine whether the regenerated message is appropriate for the user's health condition in the same manner as in operation 530. When the regenerated message is appropriate for the user's health condition, the electronic device 200 may provide the regenerated message to the user.

FIG. 6 is a flowchart illustrating a method of generating a message about health care for a user according to an embodiment.

Referring to FIG. 6, according to an embodiment, operations 610 to 650 may be performed sequentially, but not necessarily. For example, the order of operations 610 to 650 may be changed, and at least two operations may be performed in parallel. Operations 610 to 650 may be substantially the same as the operations of the state information analyzer 210 of FIG. 2 as described with reference to FIG. 2.

In operation 610, the electronic device 200 may preprocess (and/or normalize) user state information (e.g., the user state information 310 of FIG. 3). The electronic device 200 may analyze a current state (e.g., a current health condition) of a user by preprocessing the user state information 310.

In operation 630, the electronic device 200 may select a priority of the user state information 310. The electronic device 200 may select a priority for determining a topic (e.g., a topic of a health care message).

According to an embodiment, the electronic device 200 may determine whether the user state information 310 has changed, based on the preprocessed user state information. The electronic device 200 may select a priority based on a change in the user state information 310.

According to an embodiment, when the user's heart rate increases, the electronic device 200 may assign priority to a change in biometric information (e.g., the user's heart rate and/or the user's step count) to provide, with priority, a message regarding the increase in the user's heart rate. When the user's exercise state changes (e.g., when the user is exercising and/or when the user has completed exercise), the electronic device 200 may assign priority to a change in an exercise record (e.g., an activity amount, an exercise duration, and/or an amount of calories burned during exercise) to provide, with priority, an exercise-related message. When the user's sleep pattern changes, the electronic device 200 may assign priority to a change in the user's lifestyle habits (e.g., eating habits, mealtime, and/or sleep pattern) to provide, with priority, a message regarding the change in the user's sleep pattern. When a medical record changes (e.g., when the user updates a clinical result), the electronic device 200 may assign priority to a change in the medical record (e.g., an EMR and/or an EHR) to provide, with priority, a message regarding the change in the medical record. The electronic device 200 may determine the priority in the order of a change in biometric information, a change in an exercise record, a change in lifestyle habits, and a change in a medical record. However, the priority may be reselected based on user input (e.g., input regarding priority setting for generating a health-related message).

In operation 650, the electronic device 200 may determine a topic (e.g., the topic 330 of FIG. 3) of a message (e.g., the message 350 of FIG. 3). The electronic device 200 may determine one of various health-related topics (e.g., a biometric information-related topic, an exercise record-related topic, a lifestyle pattern-related topic, and/or a medical record-related topic) based on the priority to generate a message (e.g., a message about health care).

According to an embodiment, when the user's sleep time decreases and the user's exercise duration decreases, the electronic device 200 may determine an exercise record-related topic, based on the priority (e.g., the order of a change in biometric information, a change in an exercise record, a change in lifestyle habits, and a change in a medical record) to provide, with priority, an exercise-related message.

According to an embodiment, the electronic device 200 may transmit the determined topic 330 (e.g., an exercise record-related topic) to a message generator (e.g., the message generator 230 of FIG. 2). The message generator 230 may generate a message (e.g., a health-related message corresponding to a decrease in exercise duration) using a generative AI model (e.g., a generative language model), based on the topic 330 and user state information (e.g., the user state information 310 of FIG. 3) (e.g., information indicating that the user's exercise duration has decreased).

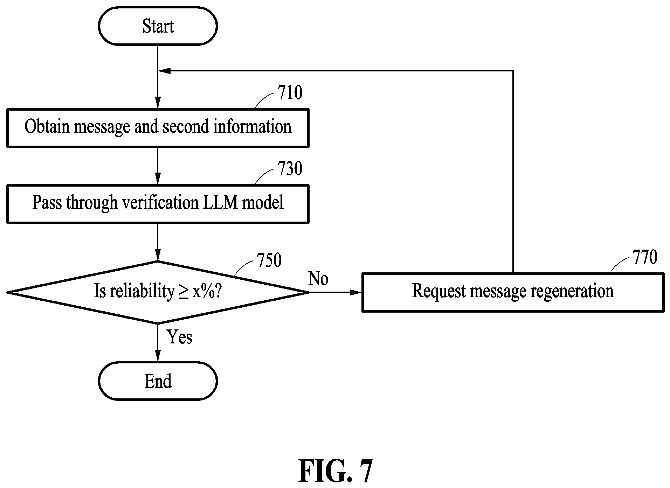

FIG. 7 is a diagram illustrating an operation of determining whether a message is appropriate for a health condition of a user, based on a reliability score, according to an embodiment.

Referring to FIG. 7, according to an embodiment, operations 710 to 770 may be performed sequentially, but not necessarily. For example, the order of operations 710 to 770 may be changed, and at least two operations may be performed in parallel.

In operation 710, the electronic device 200 may obtain (e.g., receive) a message (e.g., the message 410-1 of FIG. 4A) and second information (e.g., the second information 410-3 of FIG. 4A).

In operation 730, the electronic device 200 may calculate a reliability score of the message 410-1 using a verification LLM model (e.g., the LLM 430 of FIG. 4A), based on the message 410-1 and the second information 410-3. The reliability score may be calculated by comparing the message 410-1 with the second information 410-3. For example, the LLM 430 may include a plurality of encoder layers (e.g., Encoder layer 1 450-1 to Encoder layer N 450-N of FIG. 4A). The LLM 430 may identify the relationship, semantics, and structure of the context between the message 410-1 and the second information 410-3 through encoder layers 450. According to an embodiment, the LLM 430 may calculate a reliability score based on context information of the message 410-1 (e.g., the relationship, semantics, and structure of the context of the message 410-1) and context information of the second information 410-3 (e.g., the relationship, semantics, and structure of the context of the second information 410-3). The LLM 430 may calculate similarity between the context information of the message 410-1 and the context information of the second information 410-3. In this case, the similarity may be calculated using Euclidean distance, cosine similarity, and/or Jaccard similarity. However, the method of calculating similarity is not limited to the foregoing examples. The LLM 430 may calculate the reliability score of the message 410-1 based on similarity between the message 410-1 and the second information 410-3.

In operation 750, the electronic device 200 may compare the reliability score of the message 410-1 with a threshold (e.g., x %). The threshold may be a predetermined value. When the reliability score is less than the threshold, the electronic device 200 may perform operation 770. When the reliability score is greater than or equal to the threshold, the electronic device 200 may terminate the calculation of the reliability score of the message.

In operation 770, the electronic device 200 may request regeneration of a message. For example, when the reliability score of the message 410-1 is less than the threshold, the electronic device 200 may determine that the message 410-1 is inappropriate for the user's health condition. The electronic device 200 may output feedback (e.g., a request to regenerate a message) in response to determining that the message 410-1 is inappropriate for the user's health condition.

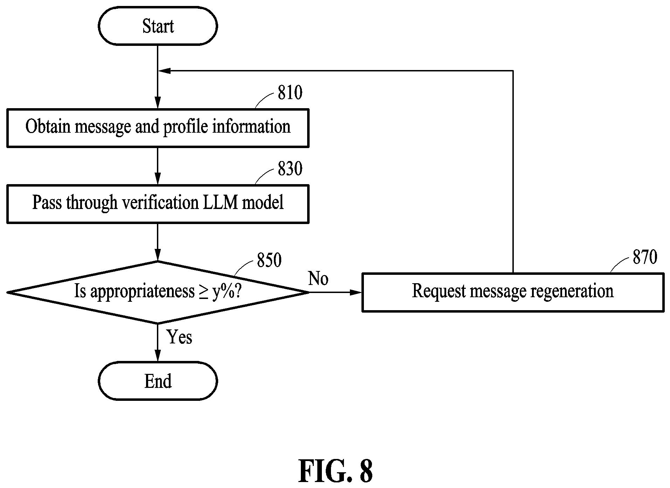

FIG. 8 is a diagram illustrating an operation of determining whether a message is appropriate for a health condition of a user, based on an appropriateness score, according to an embodiment.

Referring to FIG. 8, according to an embodiment, operations 810 to 870 may be performed sequentially, but not necessarily. For example, the order of operations 810 to 870 may be changed, and at least two operations may be performed in parallel.

In operation 810, the electronic device 200 may obtain (e.g., receive) a message (e.g., the message 410-1 of FIG. 4A) and profile information (e.g., the profile information 410-5 of FIG. 4A) of a user.

In operation 830, the electronic device 200 may calculate an appropriateness score of the message 410-1 using a verification LLM model (e.g., the LLM 430 of FIG. 4A), based on the message 410-1 and the profile information 410-5. The appropriateness score may be calculated by comparing the message 410-1 with the profile information 410-5. For example, the LLM 430 may include a plurality of encoder layers (e.g., Encoder layer 1 450-1 to Encoder layer N 450-N of FIG. 4A). The LLM 430 may identify the relationship, semantics, and structure of the context between the message 410-1 and the profile information 410-5 through the encoder layers 450. The LLM 430 may calculate an appropriateness score based on context information of the message 410-1 (e.g., the relationship, semantics, and structure of the context of the message 410-1) and context information of the profile information 410-5 (e.g., the relationship, semantics, and structure of the context of the profile information 410-5). The LLM 430 may calculate similarity between the context information of the message 410-1 and the context information of the profile information 410-5. In this case, the similarity may be calculated using Euclidean distance, cosine similarity, and/or Jaccard similarity. However, the method of calculating similarity is not limited to the foregoing examples. The LLM 430 may calculate the appropriateness score of the message 410-1 based on similarity between the message 410-1 and the profile information 410-5.

In operation 850, the electronic device 200 may compare the appropriateness score of the message 410-1 with a threshold (e.g., y %). The threshold may be a predetermined value. When the appropriateness score is less than the threshold, the electronic device 200 may perform operation 870. When the appropriateness score is greater than or equal to the threshold, the electronic device 200 may terminate the calculation of the appropriateness score of the message.

In operation 870, the electronic device 200 may request regeneration of a message. For example, when the appropriateness score of the message 410-1 is less than the threshold, the electronic device 200 may determine that the message 410-1 is inappropriate for the user's health condition. The electronic device 200 may output feedback (e.g., a request to regenerate a message) in response to determining that the message 410-1 is inappropriate for the user's health condition.

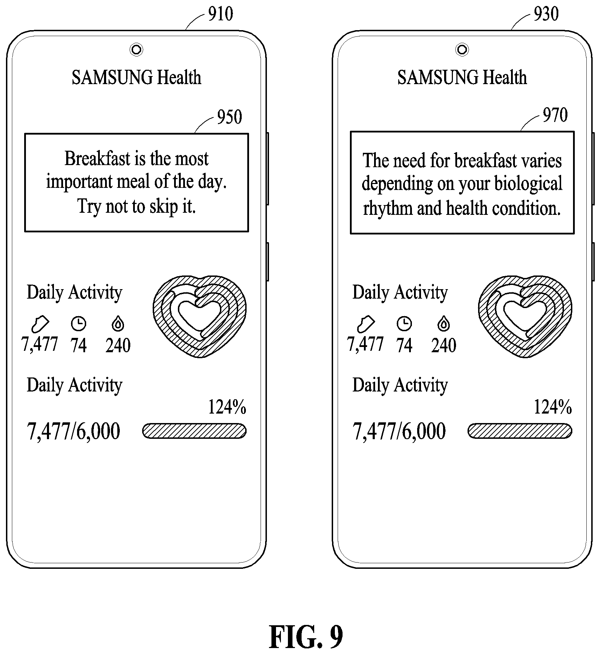

FIG. 9 is a diagram illustrating a message verified based on second information according to an embodiment.

Referring to FIG. 9, according to an embodiment, on screen 910, the message 950 may not be verified based on second information (e.g., the second information 410-3 of FIG. 4A). The message 950 may include information that is inaccurate in light of professional medical information.

According to an embodiment, on screen 930, the message 970 may be verified based on the second information (e.g., the second information 410-3 of FIG. 4A). The message 970 may include information that is accurate in light of professional medical information.

For example, eating breakfast may be beneficial to health may fall within general health knowledge. However, there may be people for whom breakfast is not beneficial to health. Information indicating that breakfast should not be skipped may be inaccurate in light of professional medical information.

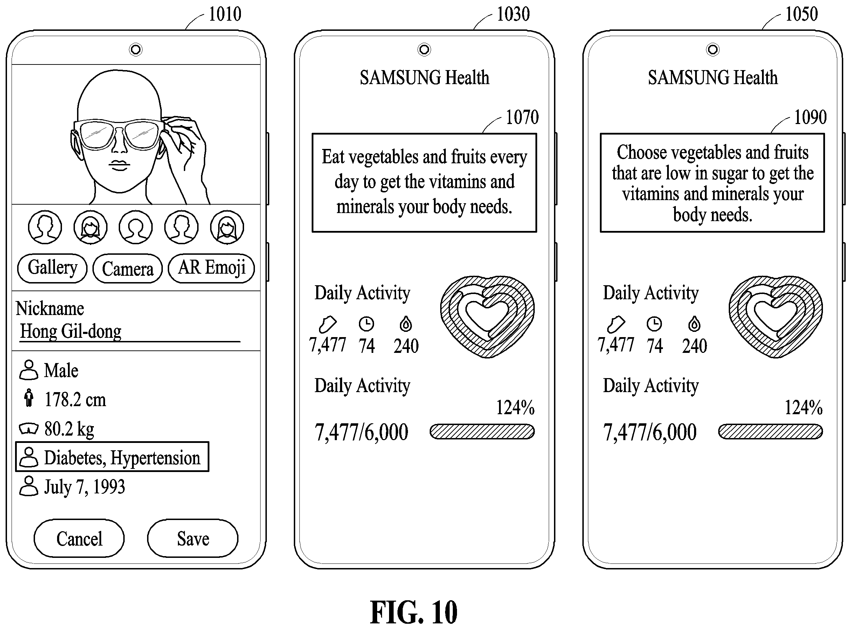

FIG. 10 is a diagram illustrating a message verified based on profile information of a user according to an embodiment.

Referring to FIG. 10, according to an embodiment, screen 1010 shows the user's profile information (e.g., the profile information 410-5 of FIG. 4A) (e.g., information indicating that a user has diabetes).

According to an embodiment, on screen 1030, a message 1070 may not be verified based on the user's profile information (e.g., information indicating that a user has diabetes). Suggesting fruit consumption to a user with diabetes may not be appropriate for the user's health condition. For example, fruits with a high sugar content may not be helpful in managing diabetes. The message 1070 suggests fruit consumption without considering the sugar content of fruit and thus may not be appropriate for the user's health condition.

According to an embodiment, on screen 1050, a message 1090 may be verified based on the user's profile information (e.g., information indicating that a user has diabetes). The message 1090 may be appropriate for the user's health condition. For example, the message 1090 may be appropriate for a user with diabetes. Suggesting consumption of fruits with a low sugar content to a user with diabetes may be appropriate for the user's health condition. In addition, the message 1090 may further provide examples of fruits that should not be consumed by the user.

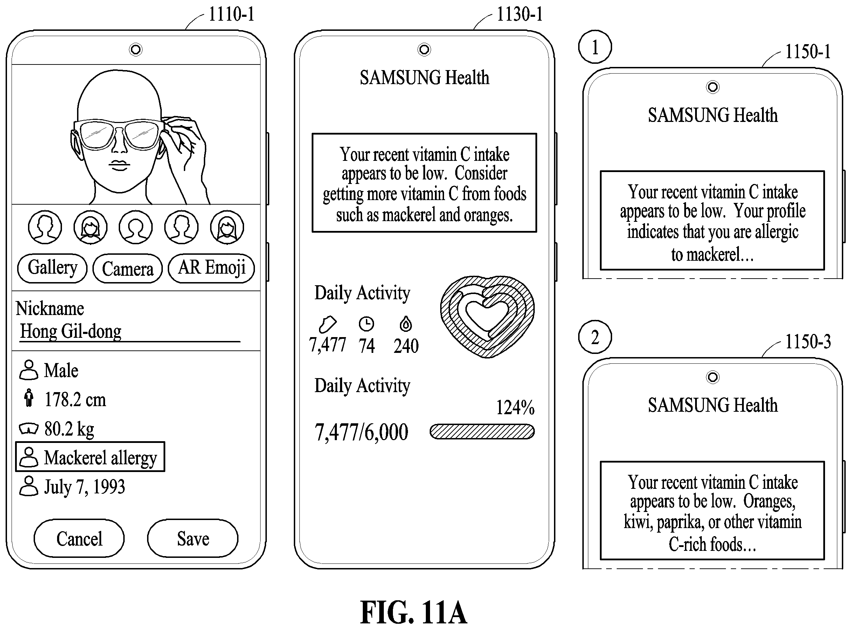

FIGS. 11A to 11C are diagrams illustrating a comparison between a verified message and an unverified message based on various profile information of a user according to an embodiment.

Referring to FIG. 11A, according to an embodiment, screen 1110-1 shows the user's profile information (e.g., the profile information 410-5 of FIG. 4A). For example, the profile information 410-5 of the user may include information indicating that the user has a mackerel allergy.

According to an embodiment, on screen 1130-1, a message may not be verified based on the profile information 410-5 of the user (e.g., information indicating that the user has a mackerel allergy). The message that is not verified based on the profile information 410-5 of the user (e.g., information indicating that the user has a mackerel allergy) may not be appropriate for the user's health condition. For example, suggesting intake of vitamin C from mackerel to a user with a mackerel allergy may not be appropriate for the user's health condition.

According to an embodiment, on screens 1150-1 and 1150-3, messages may be verified based on the profile information 410-5 of the user (e.g., information indicating that the user has a mackerel allergy). For example, the messages may include a message suggesting avoiding mackerel for vitamin C intake and/or a message suggesting intake of foods other than mackerel. The message (e.g., a message suggesting avoiding mackerel) on screen 1150-1 and the message (e.g., a message suggesting intake of foods other than mackerel) on screen 1150-3 may be appropriate for the user's health condition.

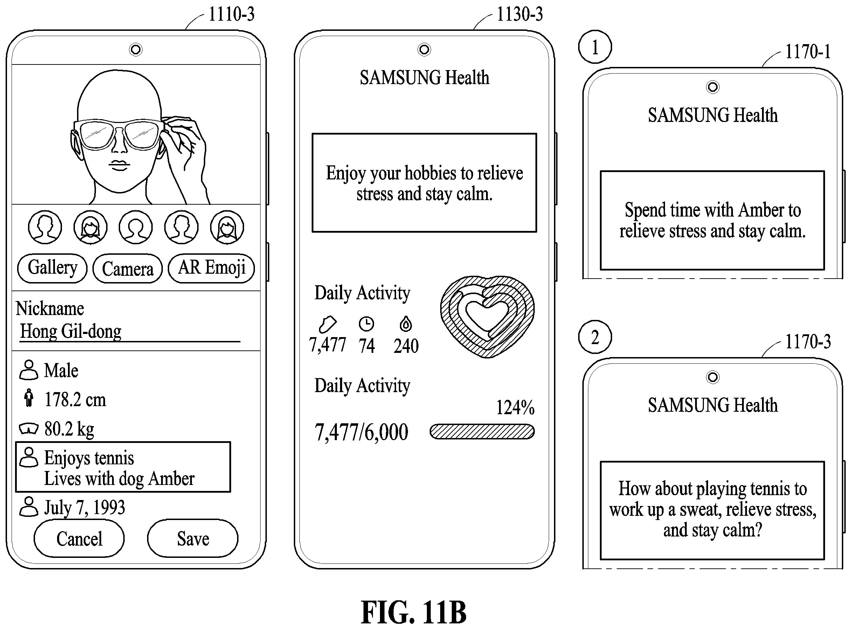

Referring to FIG. 11B, according to an embodiment, screen 1110-3 shows the profile information 410-5 of the user. For example, the profile information 410-5 of the user may include information regarding the user's hobbies. The information regarding the user's hobbies may include information indicating that the user enjoys playing tennis and/or information indicating that the user lives with a pet dog.

According to an embodiment, on screen 1130-3, a message may not be verified based on the profile information 410-5 of the user (e.g., information regarding the user's hobbies). The message may include a message in which the profile information 410-5 (e.g., information regarding the user's hobbies) is not reflected. For example, a message suggesting stress relief through hobbies may fall within general health knowledge but may not reflect information regarding specific hobbies.

According to an embodiment, on screens 1170-1 and 1170-3, messages may be verified based on the profile information 410-5 of the user (e.g., information regarding the user's hobbies). The message may include a message in which the profile information 410-5 (e.g., information regarding the user's hobbies) is reflected. For example, the message may include a message suggesting leisure activities with a pet dog and/or a message suggesting playing tennis. The message (e.g., a message suggesting leisure activities with a pet dog) on screen 1170-1 and the message (e.g., a message suggesting playing tennis) on screen 1170-3 may include a message suggesting more specific health care measures than the message (e.g., a message suggesting stress relief through hobbies) on screen 1130-3.

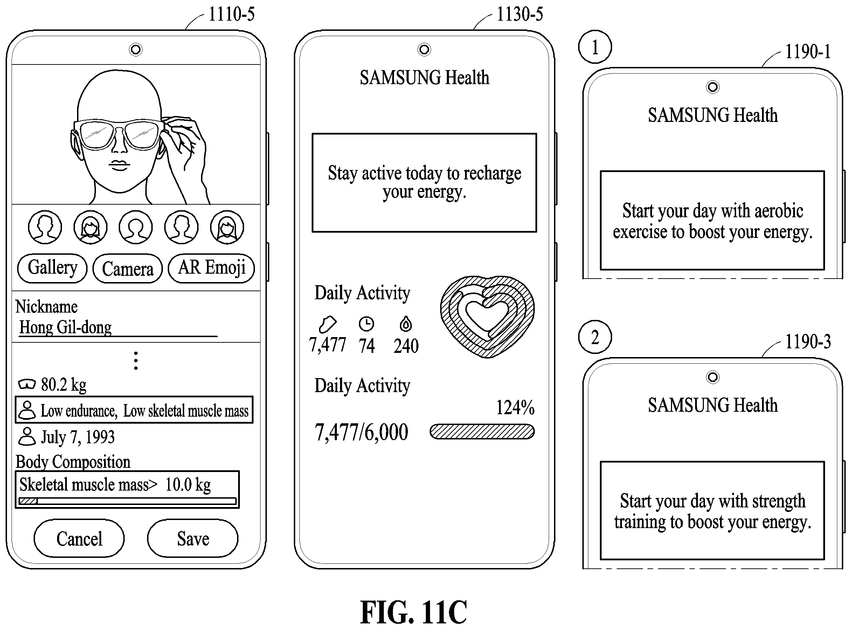

Referring to FIG. 11C, according to an embodiment, screen 1110-5 shows the profile information 410-5 of the user. For example, the profile information 410-5 of the user may include information indicating that the user has insufficient endurance and/or that the user has insufficient skeletal muscle mass.

According to an embodiment, on screen 1130-5, a message may not be verified based on the profile information 410-5 of the user (e.g., information indicating that the user has insufficient endurance and/or that the user has insufficient skeletal muscle mass). The message may include a message in which the profile information 410-5 (e.g., information indicating that the user has insufficient endurance and/or that the user has insufficient skeletal muscle mass) is not reflected. For example, a message suggesting activities for restoring energy may fall within general health knowledge but may not reflect information regarding specific activities.

According to an embodiment, on screens 1190-1 and 1190-3, messages may be verified based on the profile information 410-5 of the user (e.g., information indicating that the user has insufficient endurance and/or that the user has insufficient skeletal muscle mass). The messages may include a message in which the profile information 410-5 (e.g., information indicating that the user has insufficient endurance and/or that the user has insufficient skeletal muscle mass) is reflected. For example, the message may include a message suggesting aerobic exercise in response to insufficient endurance and/or a message suggesting strength training in response to insufficient skeletal muscle mass. The message (e.g., a message suggesting aerobic exercise in response to insufficient endurance) on screen 1190-1 and the message (e.g., a message suggesting strength training in response to insufficient skeletal muscle mass) on screen 1190-3 may include a message suggesting more specific health care measures than the message (e.g., a message suggesting activities for restoring energy) on screen 1130-5.

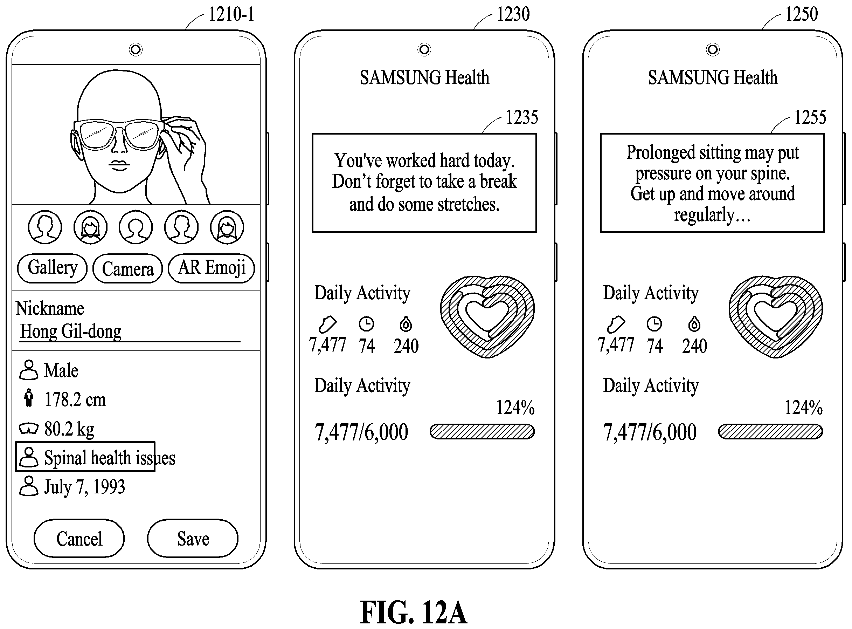

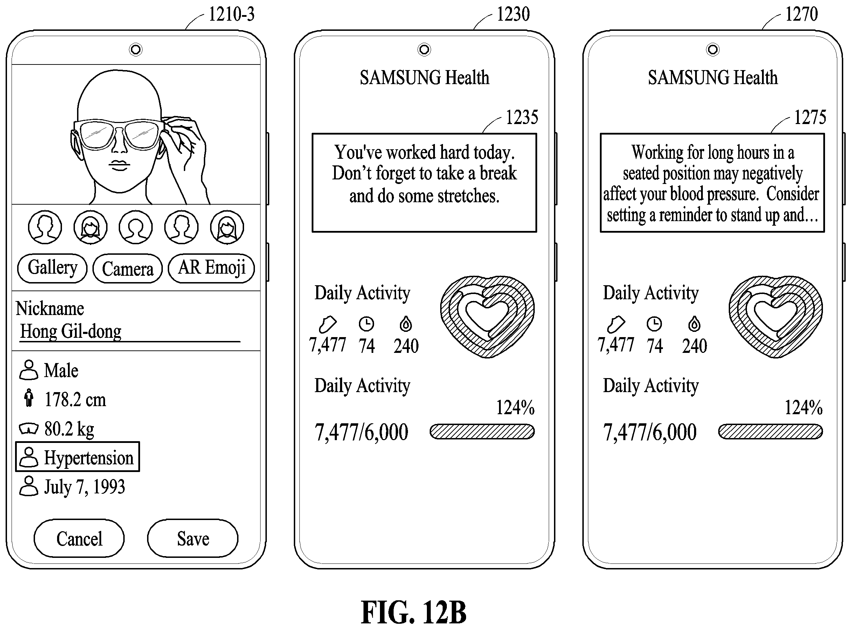

FIGS. 12A and 12B are diagrams illustrating a message through which profile information is provided to different users according to an embodiment.

Referring to FIGS. 12A and 12B, according to an embodiment, screen 1210-1 shows profile information of a first user (e.g., the profile information 410-5 of FIG. 4A) (e.g., information indicating that the first user has a spinal health issue). Screen 1210-3 shows profile information 410-5 of a second user (e.g., information indicating that the second user has high blood pressure).

According to an embodiment, on screen 1230, a message 1235 may be a message generated according to the prior art. The message 1235 may not be verified based on the profile information 410-5 of a user (e.g., the first user and the second user). Although the profile information of the first user (e.g., information indicating that the first user has a spinal health issue) is different from the profile information of the second user (e.g., information indicating that the second user has high blood pressure), the message 1235 may be provided identically to the first user and the second user.

According to an embodiment, on screens 1250 and 1270, messages 1255 and 1275 may be generated by the electronic device 200 of FIG. 2. The messages 1255 and 1275 may be verified based on different profile information 410-5. For example, the message 1255 may be verified based on the profile information of the first user (e.g., information indicating that the first user has a spinal health issue). The message 1275 may be verified based on the profile information of the second user (e.g., information indicating that the second user has high blood pressure). In other words, when a portion of the profile information 410-5 of the user is different, the electronic device 200 may generate messages (e.g., the message 1255 corresponding to a spinal health issue and the message 1275 corresponding to a high blood pressure) respectively corresponding to the different profile information (e.g., information indicating that the first user has a spinal health issue and/or information indicating that the second user has high blood pressure).

According to an embodiment, an electronic device (e.g., the electronic device 101 of FIG. 1 and the electronic device 200 of FIG. 2) may include at least one processor (e.g., the processor 120 of FIG. 1 and the processor 201 of FIG. 2) and memory (e.g., the memory 130 of FIG. 1 and the memory 203 of FIG. 2) storing instructions. The instructions, when executed individually or collectively by the at least one processor 120 and 201, may cause the electronic device 101 or 200 to generate, based on state information (e.g., the user state information 310 of FIG. 3) of a user and first information, a message (e.g., the message 350 of FIG. 3 and the message 410-1 of FIG. 4A) about health care for the user. The instructions, when executed individually or collectively by the at least one processor 120 and 201, may cause the electronic device 101 or 200 to determine, based on second information (e.g., the second information 410-3 of FIG. 4A) and profile information of the user (e.g., the profile information 410-5 of FIG. 4A), whether the messages 350 and 410-1 are appropriate for the health condition of the user. The first information may include general health-related information. The second information 410-3 may include professional health-related information.

According to an embodiment, the instructions, when executed individually or collectively by the at least one processor 120 and 201, may cause the electronic device 101 or 200 to calculate, based on the second information 410-3, a reliability score of the messages 350 and 410-1. The instructions, when executed individually or collectively by the at least one processor 120 and 201, may cause the electronic device 101 or 200 to calculate, based on the profile information 410-5 of the user, an appropriateness score of the messages 350 and 410-1.

According to an embodiment, the instructions, when executed individually or collectively by the at least one processor 120 and 201, may cause the electronic device 101 or 200 to determine, based on the reliability score and the appropriateness score, whether the messages 350 and 410-1 are appropriate for the health condition of the user.

According to an embodiment, the instructions, when executed individually or collectively by the at least one processor 120 and 201, may cause the electronic device 101 or 200 to determine, when the reliability score and the appropriateness score are greater than or equal to a threshold, that the messages 350 and 410-1 are appropriate for the health condition of the user.

According to an embodiment, the instructions, when executed individually or collectively by the at least one processor 120 and 201, may cause the electronic device 101 or 200 to provide, in response to determining that the messages 350 and 410-1 are appropriate for the health condition of the user, the messages 350 and 410-1 to the user.

According to an embodiment, the instructions, when executed individually or collectively by the at least one processor 120 and 201, may cause the electronic device 101 or 200 to determine, when the reliability score or the appropriateness score is less than the threshold, that the messages 350 and 410-1 are inappropriate for the health condition of the user.

According to an embodiment, the instructions, when executed individually or collectively by the at least one processor 120 and 201, may cause the electronic device 101 or 200 to output, in response to determining that the messages 350 and 410-1 are inappropriate for the health condition of the user, feedback for regenerating a message appropriate for the health condition of the user.

According to an embodiment, the instructions, when executed individually or collectively by the at least one processor 120 and 201, may cause the electronic device 101 or 200 to regenerate, based on the feedback, a message about health care for the user.

According to an embodiment, a method of operating an electronic device (e.g., the electronic device 101 of FIG. 1 and the electronic device 200 of FIG. 2) may include generating, based on state information (e.g., the user state information 310 of FIG. 3) of a user and first information, a message (e.g., the message 350 of FIG. 3 and the message 410-1 of FIG. 4A) about health care for the user.

The method may include determining, based on second information (e.g., the second information 410-3 of FIG. 4A) and profile information of the user (e.g., the profile information 410-5 of FIG. 4A), whether the messages 350 and 410-1 are appropriate for the health condition of the user. The first information may include general health-related information. The second information 410-3 may include professional health-related information.

According to an embodiment, the determining of whether the messages 350 and 410-1 are appropriate for the health condition of the user may include calculating, based on the second information 410-3, a reliability score of the messages 350 and 410-1.

The determining of whether the messages 350 and 410-1 are appropriate for the health condition of the user may include calculating, based on the profile information 410-5 of the user, an appropriateness score of the messages 350 and 410-1.

According to an embodiment, the determining of whether the messages 350 and 410-1 are appropriate for the health condition of the user may include determining, based on the reliability score and the appropriateness score, whether the messages 350 and 410-1 are appropriate for the health condition of the user.

According to an embodiment, the determining of whether the messages 350 and 410-1 are appropriate for the health condition of the user may include determining, when the reliability score and the appropriateness score are greater than or equal to a threshold, that the messages 350 and 410-1 are appropriate for the health condition of the user.

According to an embodiment, the determining that the messages 350 and 410-1 are appropriate for the health condition of the user may further include providing, in response to determining that the messages 350 and 410-1 are appropriate for the health condition of the user, the messages 350 and 410-1 to the user.

According to an embodiment, based on the reliability score and the appropriateness score, the determining of whether the messages 350 and 410-1 are appropriate for the health condition of the user may include determining, when the reliability score or the appropriateness score is less than the threshold, that the messages 350 and 410-1 are inappropriate for the health condition of the user.

According to an embodiment, the determining that the messages 350 and 410-1 are inappropriate for the health condition of the user may further include outputting, in response to determining that the messages 350 and 410-1 are inappropriate for the health condition of the user, feedback for regenerating a message appropriate for the health condition of the user.

According to an embodiment, the outputting of the feedback may further include regenerating, based on the feedback, a message about health care for the user.

According to an embodiment, an electronic device (e.g., the electronic device 101 of FIG. 1 and the electronic device 200 of FIG. 2) may include at least one processor (e.g., the processor 120 of FIG. 1 and the processor 201 of FIG. 2) and memory (e.g., the memory 130 of FIG. 1 and the memory 203 of FIG. 2) storing instructions. The instructions, when executed individually or collectively by the at least one processor 120 and 201, may cause the electronic device 101 or 200 to generate, based on state information (e.g., the user state information 310 of FIG. 3) of a user and health-related information (e.g., first information), a message (e.g., the message 350 of FIG. 3 and the message 410-1 of FIG. 4A) about health care for the user. The instructions, when executed individually or collectively by the at least one processor 120 and 201, may cause the electronic device 101 or 200 to determine, based on profile information of the user (e.g., the profile information 410-5 of FIG. 4A), whether the messages 350 and 410-1 are appropriate for the health condition of the user.

According to an embodiment, the instructions, when executed individually or collectively by the at least one processor 120 and 201, may cause the electronic device 101 or 200 to calculate, based on the profile information of the user, a reliability score of the messages 350 and 410-1. The instructions, when executed individually or collectively by the at least one processor 120 and 201, may cause the electronic device 101 or 200 to determine, based on the reliability score, whether the messages 350 and 410-1 are appropriate for the health condition of the user.

According to an embodiment, the instructions, when executed individually or collectively by the at least one processor 120 and 201, may cause the electronic device 101 or 200 to provide, in response to determining that the messages 350 and 410-1 are appropriate for the health condition of the user, the message to the user.

According to an embodiment, the instructions, when executed individually or collectively by the at least one processor 120 and 201, may cause the electronic device 101 or 200 to output, in response to determining that the messages 350 and 410-1 are inappropriate for the health condition of the user, feedback for regenerating a message appropriate for the health condition of the user. The instructions, when executed individually or collectively by the at least one processor 120 and 201, may cause the electronic device 101 or 200 to regenerate, based on the feedback, a message appropriate for the health condition of the user.

The effects to be achieved are not limited to those described above, and other effects not mentioned above will be clearly understood by one of ordinary skill in the art from the description of the present disclosure.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment, the electronic device is not limited to those described above.

It should be understood that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related components. It is to be understood that a singular form of a noun corresponding to an item may include one or more of the things, unless the relevant context clearly indicates otherwise. As used herein, "A or B," "at least one of A and B," "at least one of A or B," "A, B or C," "at least one of A, B and C," and "at least one of A, B, or C," each of which may include any one of the items listed together in the corresponding one of the phrases, or all possible combinations thereof. Terms such as "1st" and "2nd," or "first" and "second" may be used to simply distinguish a corresponding component from other components, and do not limit the components in other aspects (e.g., importance or order). It is to be understood that if a component (e.g., a first component) is referred to, with or without the term "operatively" or "communicatively," as "coupled with," "coupled to," "connected with," or "connected to" another component (e.g., a second component), the component may be coupled with the other component directly (e.g., by wire), wirelessly, or via a third component.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, the module may be implemented in the form of an application-specific integrated circuit (ASIC).

Various embodiments as set forth herein may be implemented as software (e.g., the program 140) including one or more instructions that are stored in a storage medium (e.g., the internal memory 136 or the external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as a memory of the manufacturer's server, a server of the application store, or a relay server.

According to an embodiment, each component (e.g., a module or a program) of the above-described components may include a single entity or multiple entities, and some of the multiple entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101 and 200) comprising:
at least one processor (120 and 201); and
memory (130 and 203) storing instructions,
wherein the instructions, when executed individually or collectively by the at least one processor (120 and 201), cause the electronic device (101 and 200) to:
generate, based on state information (310) of a user and first information, a message (350 and 410-1) about health care for the user; and
determine, based on second information (410-3) and profile information (410-5) of the user, whether the message (350 and 410-1) is appropriate for a health condition of the user,
wherein the first information includes general health-related information, and
the second information (410-3) includes professional health-related information.

2. The electronic device (101 and 200) of claim 1, wherein the instructions, when executed individually or collectively by the at least one processor (120 and 201), cause the electronic device (101 and 200) to:
calculate, based on the second information (410-3), a reliability score of the message (350 and 410-1); and
calculate, based on the profile information (410-5) of the user, an appropriateness score of the message (350 and 410-1).

3. The electronic device (101 and 200) of any one of claims 1 and 2, wherein the instructions, when executed individually or collectively by the at least one processor (120 and 201), cause the electronic device (101 and 200) to:
determine, based on the reliability score and the appropriateness score, whether the message (350 and 410-1) is appropriate for a health condition of the user.

4. The electronic device (101 and 200) of any one of claims 1 to 3, wherein the instructions, when executed individually or collectively by the at least one processor (120 and 201), cause the electronic device (101 and 200) to:
determine, when the reliability score and the appropriateness score are greater than or equal to a threshold, that the message (350 and 410-1) is appropriate for a health condition of the user.

5. The electronic device (101 and 200) of any one of claims 1 to 4, wherein the instructions, when executed individually or collectively by the at least one processor (120 and 201), cause the electronic device (101 and 200) to:
provide, in response to determining that the message (350 and 410-1) is appropriate for a health condition of the user, the message (350 and 410-1) to the user.

6. The electronic device (101 and 200) of any one of claims 1 to 5, wherein the instructions, when executed individually or collectively by the at least one processor (120 and 201), cause the electronic device (101 and 200) to:
determine, when the reliability score or the appropriateness score is less than the threshold, that the message (350 and 410-1) is inappropriate for a health condition of the user.

7. The electronic device (101 and 200) of any one of claims 1 to 6, wherein the instructions, when executed individually or collectively by the at least one processor (120 and 201), cause the electronic device (101 and 200) to:
output, in response to determining that the message (350 and 410-1) is inappropriate for a health condition of the user, feedback for regenerating a message appropriate for the health condition of the user.

8. The electronic device (101 and 200) of any one of claims 1 to 7, wherein the instructions, when executed individually or collectively by the at least one processor (120 and 201), cause the electronic device (101 and 200) to:
regenerate, based on the feedback, a message about health care for the user.

9. A method of operating an electronic device (101 and 200), the method comprising:
generating, based on state information (310) of a user and first information, a message (350 and 410-1) about health care for the user; and
determining, based on second information (410-3) and profile information (410-5) of the user, whether the message (350 and 410-1) is appropriate for a health condition of the user,
wherein the first information includes general health-related information, and
the second information (410-3) includes professional health-related information.

10. The method of claim 9, wherein the determining of whether the message (350 and 410-1) is appropriate for a health condition of the user comprises:
calculating, based on the second information (410-3), a reliability score of the message (350 and 410-1); and
calculating, based on the profile information (410-5) of the user, an appropriateness score of the message (350 and 410-1).

11. The method of any one of claims 9 and 10, wherein the determining of whether the message (350 and 410-1) is appropriate for a health condition of the user comprises:
determining, based on the reliability score and the appropriateness score, whether the message (350 and 410-1) is appropriate for a health condition of the user.

12. The method of any one of claims 9 to 11, wherein, based on the reliability score and the appropriateness score, the determining of whether the message (350 and 410-1) is appropriate for a health condition of the user comprises:
determining, when the reliability score and the appropriateness score are greater than or equal to a threshold, that the message (350 and 410-1) is appropriate for a health condition of the user.

13. The method of any one of claims 9 to 12, wherein the determining that the message (350 and 410-1) is appropriate for a health condition of the user further comprises:
providing, in response to determining that the message (350 and 410-1) is appropriate for a health condition of the user, the message (350 and 410-1) to the user.

14. The method of any one of claims 9 to 13, wherein, based on the reliability score and the appropriateness score, the determining of whether the message (350 and 410-1) is appropriate for a health condition of the user comprises:
determining, when the reliability score or the appropriateness score is less than the threshold, that the message (350 and 410-1) is inappropriate for a health condition of the user.

15. The method of any one of claims 9 to 14, wherein the determining that the message (350 and 410-1) is inappropriate for a health condition of the user further comprises:
outputting, in response to determining that the message (350 and 410-1) is inappropriate for a health condition of the user, feedback for regenerating a message appropriate for the health condition of the user.
